# EUROPÄISCHE PATENTSCHRIFT

(11) **EP 0 338 971 B1**
(45) Veröffentlichungstag und Bekanntmachung des Hinweises auf die Patenterteilung: **20.01.1993**
(21) Anmeldenummer: 89730103.2
(22) Anmeldetag: 13.04.1989
(51) Int. Cl.: A61K 9/50, A61K 47/00

(54) **Verfahren zur Herstellung von Liposomen und/oder biologischer Zellen, die Inhaltsstoffe eingeschlossen haben, sowie deren Verwendung**
Process for preparing liposomes and/or biological cells containing ingredients, and their use
Procédé pour la préparation de liposomes et/ou de cellules biologiques contenant des ingrédients et leur utilisation

(30) Priorität: 16.04.1988 DE 3812816
(43) Veröffentlichungstag der Anmeldung: 25.10.1989
(73) Patentinhaber: SCHERING AKTIENGESELLSCHAFT, 13342 Berlin (DE)
(72) Erfinder: Lawaczeck, Rüdiger, Dr., D-1000 Berlin 27 (DE)

(56) Entgegenhaltungen:
- EP-A- 0 055 576
- EP-A- 0 069 307
- WO-A-86/00238
- FR-A- 2 373 289
- US-A- 4 327 710

## Beschreibung

Die Erfindung betrifft den in den Patentansprüchen genannten Gegenstand, d.h. ein Verfahren zur Herstellung von Liposomen und/oder biologischer Zellen, die während der Herstellung extern zugesetzte Substanzen aufnehmen. In beiden Fällen erfolgt die Substanzaufnahme unter schonenden Bedingungen. Die beladenen Liposomen und/oder Zellen sind als zielgerichtete Pharmakaträger geeignet.

Liposomen sind aus Lipidmolekülen, vorzugsweise Phospholipiden, aufgebaut. Diese bilden im wässrigen Medium spontan Lipiddoppelschichten aus, die geschlossen und lamellenartig angeordnet sind. Eine oder mehrere Lamellen trennen das innere Kompartment von der äußeren Lösung. Die polaren hydrophilen Kopfgruppen der Lipide (z.B. Ethanolamin beim Cephalin, Cholin beim Lecithin) zeigen zur Wasserphase hin, die unpolaren hydrophoben Fettsäurereste sind ins Innere der Lipiddoppelschicht gerichtet. Die Doppelschichten sind zwei Moleküllagen oder 4 bis 5 nm dick. Man unterscheidet die Liposomen nach ihrer Größe und Anzahl der Lamellen und spricht von kleinen, unilamellaren (small unilamellar vesicles (SUV)) mit Radien bis zu 100 nm, großen unilamellaren (large unilamellar vesicles (LUV)) mit Radien größer als 100 nm sowie multilamellaren Liposomen (multilamellar vesicles (MLV)). In wässriger Phase lagern sich Phospholipide ohne Zutun zu multilamellaren Liposomen zusammen, bei denen zwiebelartig mehrere Lipiddoppelschichten durch Wasserschichten getrennt angeordnet sind.

Die Ausbildung lamellar angeordneter Lipiddoppelschichten ist eine Folge der Balance physikalischer Kräfte und sterischer Effekte. Die Lipiddoppelschicht formt auch den Bildungsblock der Plasmamembranen, die biologische Zellen umhüllen und bei denen als zweiter Baustein Proteine in die Lipidmatrix eingelagert sind.

Die physikalischen und physiologischen Eigenschaften von Liposomen sind durch deren Lipidzusammensetzung, Größe, Temperatur, pH, Art und Stärke des Puffers, Herstellungsbedingungen, Historie und Alter bestimmt. Die Verwendung von Liposomen, die mit Arzneimitteln beladen wurden, hat in der Diagnostik und Therapie großes Interesse gefunden. Liposomen eröffnen als zielgerichtete Träger von Pharmaka neue Diagnose- und Therapieformen (s. z.B. R. Lawaczeck "Liposomen als zielgerichtete Pharmakaträger", Deutsche Apotheker Zeitung 127, 1771- 1773 (1987), M.J. Ostro "Liposomes", Sci. Am. 256, 90-99(1987)). Für pharmazeurische Zwecke sind die Permeabilität der Lipidmembran und die Stabilität der Liposomen von Interesse; physiolgisch ist zudem die Fusogenität und die Kinetik der Zellaufnahme wichtig.

Aufgrund des breiten Interesses gibt es eine Vielzahl von Veröffentlichungen, die sich mit der Physik, Chemie, Biologie, Herstellung und Verwendung von Liposomen befassen. Ein kürzlich erschienener Übersichtsartikel mit pharmazeutischer Ausrichtung wurde von D. Lichtenberg und Y. Barenholz ("Liposomes: preparation, characterization, and preservation" in Methods of Biochemical Analysis Vol. 33, 337-462 (1988)) publiziert. Die Verwendung von Zellen als Pharmakaträger wurde u.a. von C. Nicolau und Mitarbeitern beschrieben ("Resealed red blood cells as a new blood transfusion product", Biblthca haemat. 51, 82-91 (1985)).

Zur Herstellung uni- und oligolamellarer sowie beladener Liposomen wurden verschiedene Strategien entwickelt (referiert bei Lichtenberg & Barenholz), die schematisch klassifiziert werden können als 1. physikalische Zerschlagung durch Scherkräfte und 2. chemische Desintegration durch "Helfermoleküle" der sich spontan bildendenden multilamellaren Liposomen. Die Anwendung von Scherkräften ist beim Einschluß labiler Substanzen nicht geeignet. Helfermoleküle stehen in Form von Detergentien oder organischer Lösungsmittelmoleküle zur Verfügung. Mit deren Hilfe werden Nicht-Doppelschicht-Aggregate der Lipidmoleküle (meist Mizellen) ausgebildet. Das Entfemen der Helfermoleküle führt wieder zu den sich spontan bildenden liposomalen Doppelschichten und zur gleichzeitigen Aufnahme zugesetzter Substanzen. Die vollständige Entfernung der Detergenzmoleküle ist oft schwierig, zudem kann bei proteinhaltigen Einschlußmolekülen eine ungewünschte Denaturierung hinderlich sein. Die Aufnahme durch biologische Zellen kann durch hypo-osmolares Aufbrechen (Lyse) der Zellen mit nachfolgender Einstellung der Isotonie oder durch elektrisch induzierte Porenbildung erreicht werden. Dabei werden extern vorhandene Substanzen aufgenommen und während des Verschließens der Membran verkapselt. Eine schonende Lyse unter isotonen, detergenz- und spannungsfreien Bedingungen ist bisher nicht bekannt. Es besteht demnach ein Bedarf an einem Herstellungsverfahren für inhalustoffe-enthaltende Liposomen und/oder biologische Zellen, das die obengenannten Nachteile nicht aufweist. Diese Aufgabe wird durch die vorliegende Erfindung gelöst.

Es wurde nun gefunden, daß eine liposomale und/oder zelluläre Aufnahme von Substanzen, vorzugsweise Pharmaka für therapeutische und diagnostische Zwecke, überraschenderweise auch durch die Anwendung gasförmiger Helfermoleküle erreicht werden kann. Dieses Verfahren weist nicht die Mängel der oben skizzierten Herstellungsverfahren auf. Bei diesem neuen Verfahren wird die wäßrige Lipid- oder Zellsuspension, die neben den Lipiden oder Zellen die einzuschließende Substanz enthält, erfindungsgemäß mit einem Gas versetzt. Es kommen die induspruch 1 genannten Gase mit "detergenzähnlichem Effekt" infrage, d.h. Gase, die im Gegensatz zum hydrostarischen Druck die Ordnung der Lipidmoleküle mit steigendem Druck emiedrigen und/oder zu einer Solubilisierung der Membran führen. Es wird bei dem jeweils angewendeten Druck die Einstellung des thermodynamischen Gleichgewichts mit der wäßrigen Lipid- oder Zellsuspension abgewartet. Die verwendeten Gase müssen chemisch hinsichtlich der Lipide und der Wasserphase inert sein und nicht wie Kohlendioxid oder Ammoniak eine Wechelwirkung mit Wasser eingehen. Ein Überschreiten der Siedekurve im p, T-Diagramm in den flüssigen Zustand kann vorteilhaft sein. Die beim Verfahren einguretzenden Gase sind Lachgas (N₂0), Halothan, Argon, Xenon, Schwefelhexafluorid (SF₆), Alkane wie Methan, Ethan, Propan, Butan, Hexan, Heptan, Alkene wie Ethen, Propen, Buten, Hexen, Hepten. Diese Gase sind leicht handhabbar, stehen, technisch zur Verfügung, hinterlassen in einem Druckbereich bis zu 1000 bar, vorzugsweise bis zu 200 bar keinen toxischen Rückstand. Bei Lachgas wird ein Druck bis 55 bar gewählt, wie er in technischen Druckflaschen zur Verfügung steht. Für die Temperatur steht der Bereich zwischen dem jeweiligen Gefrier- und Siedepunkt zur Verfügung, vorzugsweise 0° bis 37° C. Die genannten Gasmoleküle üben einen "detergenzähnlichen" Effekt aus und führen zu einer Öffnung und/oder Solubilisierung der Lipiddoppelschicht und/oder Membran. Während der Entspannungsphase bilden sich spontan neue Liposomen aus bzw. verschließen die Membranen wieder, so daß die zugesetzten Substanzen aufgenommen sind. Dieser Prozeß kann zyklisch wiederholt werden. Bei Liposomen ist eine Einengung der Liposomengröße über eine Druckentspannung auf Atmosphärendruck durch größenselektive Trennfilter möglich. Die Lipidzusammensetzung der Liposomen kann je nach therapeutischem und/oder diagnostischem Nutzen und nach dem Zielort optimiert werden. Bei den biologischen Zellen kommen neben den Zellen der Blutbahn wie Erythrocyten, Leukocyten, Lymphocyten und Thrombocyten vorzugsweise Zellen, die in Kultur gehalten werden, infrage.

Die in WO-A-8600238 beschriebene Extrusionstechnik zur Herstellung von Liposomen verwendet ein inertes Gas (Stickstoff, das für das erfindungsgemäße Verfahren nicht geeignet ist) einzig zur Erzeugung eines hydrostatischen Drucks, um die Lipidsuspension durch geeignete Filter zur Einengung der Liposomengröße und Zahl der Doppelschichtlamellen zu drücken. Es wird kein Hinweis auf aufzunehmende Inhaltsstoffe gegeben.

Das in EP-A-0069307 beschriebene Verfahren zur Herstellung von Liposomen ist dadurch gekennzeichnet, daß man in einer wäßrigen Lösung eines Liposomenbildners in Gegenwart eines inerten flüchtigen organischen Lösungsmittels oder eines Gemischs solcher Lösungsmittel oder eines inerten anorganischen oder organischen Gases durch Schwankungen des lokalen Drucks (vorzugsweise durch Ultraschall) eine Phasengrenze gegenüber der wäßrigen Phase erzeugt.

Im Gegensatz zu diesen beiden Verfahren kommen im erfindungsgemäßen Verfahren nur Gase mit "detergenzähnlichem Effekt" infrage, d.h. Gase, die im Gegensatz zum hydrostatischen Druck die Ordnung der Lipidmoleküle mit steigendem Druck erniedrigen und/oder zu einer Solubilisierung der Membran und damit zur Aufhebung der Phasengrenze führen. Ein Hinweis auf die zelluläre Verkapselung von Inhaltsstoffen fehlt in beiden Patentanmeldungen.

Die Vorteile der Liposomenherstellung und Zellbeladung mit dem "Gashelfer-Verfahren" liegen auf der Hand:
Das Verfahren ist technisch leicht zu realisieren und kostengünstig.
Die Gasmoleküle werden im Gegensatz zu den herkömmlichen Detergenzmolekülen leicht entfernt.
Wegen des inerten Charakters und der nur in Spuren zurückbleibenden Mengen spielt die Toxizität der verwendeten Moleküle - wenn überhaupt - eine untergeordnete Rolle.
Vorteilhaft ist das Arbeiten bei physiologischen Temperaturen oder darunter.
Die Anwendung der Inertgase in dem genannten Druckbereich ist nicht schädigend für die meisten Substanzen oder Zellen.

Das Verfahren ist zur liposomalen und zellulären Addition, Inkorporation und/oder Substitution hydro- und lipophiler Substanzen geeignet.

Die beladenen Liposomen können oberflächenmodifiziert und als ziel- oder organspezifische Pharmakaträger oder Pharmakaspeicher verwendet werden.

### Beispiele

### Beispiel 1:

20 mg Dimyristoyl-Lecithin werden in 1 ml Chloroform aufgenommen. Die Lösung wird in das Druckgefäß gegeben. Das Chloroform wird mit nachgereinigtem Stickstoff und nachfolgend unter Vakuum abgedampft. 2 ml Wasser enthaltend 1 mM Carboxyfluorescein (gereinigt und aus Ethanol umkristallisiert), 20 mM Tris HCI auf pH 8 eingestellt, werden eingefüllt. CO₂ und O₂ werden durch Spülen mit Lachgas ausgetrieben. Das Druckgefäß wird verschlossen und ein Lachgasdruck von 50 bar bei Raumtemperatur angelegt. Die Lösung wird 0.5 Stunden geschüttelt, danach wird langsam und isotherm auf Atmosphärendruck entspannt. Der Vorgang wird 5 mal wiederholt. Die Lösung wird aus dem Druckgefäß genommen und zur Entfernung des extern vorhandenen Carboxyfluoresceins über einen Anionenaustauscher gegeben. Die Liposomenfraktion enthält den eingeschlossenen Farbstoff und kann für Fluoreszenzstudien eingesetzt werden.

### Beispiel 2:

wie Beispiel 1, anstelle von Dimyristoyl-Lecithin werden 200 mg einer 4 : 1 Mischung aus Eigelblecithin und Cholesterin genommen. Carboxyfluorescein wird durch 20 mM Gadolinium-DTPA als MR-Kontrastmittel ersetzt. Die erhaltenen Liposomen können in der MR-Diagnostik eingesetzt werden.

### Beispiel 3:

Herstellung von Erythrocyten oder Erythrocyten-Ghosts, die mit Carboxyfluorescein beladen sind, unter blurisotonen Bedingungen.
Frisches Rinderblut wird 3 mal in PBS Puffer pH 7.4 gewaschen und bis auf die Erythrocyten von anderen Bestandteilen befreit. Die dunkelrote, trübe Erythrocytensuspension wird mit PBS Puffer, der Carboxyfluorescein enthält, verdünnt, so daß die Konzentration des Carboxyfluoresceins 10⁻⁵ M beträgt. Die Lösung wird in eine Druckmeßzelle gegeben. Nach Spülen mit N₂O werden 50 bar N₂O bei 37° C angelegt, gerührt und die optische Dichte bei 675 nm verfolgt. Zwischen 400 und 600 min tritt Lyse der Zellen ein, deutlich sichtbar durch den typisch sigmoiden Abfall der optischen Dichte. Referenzmessungen unter Atmosphärendruck oder 50 bar hydrostatischem Druck zeigen bis zu 18 Stunden keine Lyse der Erythrocyten. Die Lösungen werden langsam auf Atmosphärendruck entspannt und unter dem Mikroskop in Durchsicht und Fluoreszenz untersucht. Die Erythrocyten zeigen vor und nach Lyse das typisch discoide Aussehen. Nicht-lysierte Zellen (Kontrollen) enthalten im Gegensatz zu den zwischenzeitlich lysierten kein Carboxyfluorescein. Die Zellfluoreszenz ist auch nach extensivem Waschen unverändert sichtbar. Es ist damit offensichtlich, daß die Erythrocyten den Farbstoff im lysierten Zustand unter isotonen Bedingungen aufgenommen haben und während der Entspannung wieder versiegelt wurden.

### Beispiel 4:

Wie Beispiel 3, nur werden Schaferythrocyten anstelle der Rindererythrocyten in 2 mM KH₂PO₄, 9 mM Na₂HPO₄, 3 mM KCl, 137 mM NaCl pH 7.5 verwendet. Die Lyse tritt zwischen 55 bis 70 min ein. Die Ergebnisse sind mit denen aus Beispiel 3 vergleichbar.

## Patentansprüche

1. Verfahren zur Herstellung inhaltsstoffe-enthaltender Liposomen und/oder biologischer Zellen, dadurch gekennzeichnet, daß Liposomen oder biologische Zellen in wäßrigem Medium mit Lachgas (N₂O), Halothan, Argon, Xenon, Schwefelhexafluorid (SF₆), Alkanen wie Methan, Ethan, Propan, Butan, Hexan, Heptan, Alkenen wie Ethen, Propen, Buten, Hexen, Hepten und/oder deren Mischungen, die mit steigendem Druck zu einer zunehmenden Abnahme der Lipidordnung führen, im gasförmigen oder flüssigen Zustand unter Zusatz der gewunschten Inhaltsstoffe unter Druck behandelt werden und anschließend eine Druckentspannung auf Atmosphärendruck vorgenommen wird.

2. Verfahren nach Anspruch 1, dadurch gekennzeichnet, daß die von den Liposomen oder biologischen Zellen aufgenommenen Inhaltsstoffe Pharmaka sind.

3. Verfahren nach Anspruch 1, dadurch gekennzeichnet, daß ein Druck bis zu 1000 bar, vorzugsweise bis zu 200 bar, verwendet wird.

4. Verfahren nach Anspruch 1, dadurch gekennzeichnet, daß zur Druckentspannung die Liposomensuspension durch größenselektive Filter gedrückt wird.

5. Verfahren nach Anspruch 1, dadurch gekennzeichnet, daß die Inhaltsstoffe inkorporiert, addiert und/oder substituiert werden.

6. Verfahren nach Anspruch 1, dadurch gekennzeichnet, daß die biologischen Zellen Zellen der Blutbahn wie Erythrocyten, Leucocyten, Lymphoryten und Thrombocyten sind.

7. Verfahren nach Anspruch 1, dadurch gekennzeichnet, daß die biologischen Zellen Zellen sind, die in Kultur gehalten werden und die pflanzlichen und/oder tierischen und/oder mikrobiellen Ursprungs sind.

8. Verwendung der nach Anspruch 1 hergestellten Liposomen und/oder biologischen Zellen für die Herstellung von Vehikeln für den Transport und/oder Speicherung von Pharmaka für diagnostische und/oder therapeutische Zwecke unter Ausschluß von Verfahren gemäß Artikel 52(4) EPÜ.

## Claims

1. Process for the preparation of liposomes and/or biological cells containing inclusion substances, characterised in that liposomes or biological cells in an aqueous medium, with the addition of the desired inclusion substances, are treated under pressure with nitrous oxide (N₂O), halothane, argon, xenon, sulphur hexafluoride (SF₆), alkanes such as methane, ethane, propane, butane, hexane or heptane, alkenes such as ethene, propene, butene, hexene or heptene, and/or mixtures thereof, in the gaseous or liquid state, which with increasing pressure lead to an increasing reduction in the lipid arrangement, and then a discharge of pressure to atmospheric pressure is carried out.

2. Process according to claim 1, characterised in that the inclusion substances absorbed by the liposomes or biological cells are pharmaceutical agents.

3. Process according to claim 1, characterised in that a pressure of up to 1000 bar, preferably up to 200 bar, is used.

4. Process according to claim 1, characterised in that for the purpose of pressure discharge, the liposome suspension is pressed through size-selective filters.

5. Process according to claim 1, characterised in that the inclusion substances are incorporated, added and/or substituted.

6. Process according to claim 1, characterised in that the biological cells are cells of the blood stream, such as erythrocytes, leucocytes, lymphocytes or platelets.

7. Process according to claim 1, characterised in that the biological cells are cells which are maintained in culture and which are of plant and/or animal and/or microbial origin.

8. Use of the liposomes and/or biological cells prepared according to claim 1 for the preparation of vehicles for transporting and/or storing pharmaceutical agents for diagnostic and/or therapeutic purposes, with the exception of processes according to Article 52(4) EPC.

## Revendications

1. Procédé de préparation de liposomes et/ou de cellules biologiques renfermant des matières d'inclusion, procédé caractérisé en ce que l'on ajoute sous pression aux liposomes ou aux cellules en milieu aqueux l'un des gaz suivants : gaz hilarant (N₂O), halothane, argon, xénon, hexafluorure de soufre (SF₆), alcanes tels que méthane, éthane, propane, butane, hexane ou heptane, alcènes tels que éthène, propène, butène, hexène ou heptène, ou un mélange de plusieurs de ces gaz, qui produisent avec une élévation de pression une diminution croissante de l'ordre des lipides, à l'état gazeux ou liquide, ainsi que les matières d'inclusion voulues, puis on détend la pression jusqu'à la pression atmosphérique.

2. Procédé selon la revendication 1, caractérisé en ce que les matières d'inclusion prises par les liposomes ou les cellules biologiques sont des produits pharmaceutiques.

3. Procédé selon la revendication 1, caractérisé en ce que l'on applique une pression pouvant s'élever jusqu'à 1000 bar, de préférence jusqu'à 200 bar.

4. Procédé selon la revendication 1, caractérisé en ce que pour la détente de pression on fait passer la suspension des liposomes sous pression à travers des filtres très sélectifs.

5. Procédé selon la revendication 1, caractérisé en ce que les matières d'inclusion sont incorporées ou ajoutées et/ou sont des matières de remplacement.

6. Procédé selon la revendication 1, caractérisé en ce que les cellules biologiques sont des cellules sanguines telles que érythrocytes, leucocytes, lymphocytes et thrombocytes.

7. Procédé selon la revendication 1, caractérisé en ce que les cellules biologiques sont des cellules maintenues en culture, d'origine végétale et/ou animale et/ou microbienne.

8. L'emploi des liposomes et/ou des cellules biologiques qui ont été obtenus selon la revendication 1 en vue d'en former des véhicules pour le transport et/ou des réserves de produits pharmaceutiques à des fins diagnostiques et/ou thérapeutiques, à l'exclusion des méthodes qui sont indiquées à l'article 52(4) de la convention sur les brevets européens.
